**Europäisches Patentamt**

**European Patent Office**

**Office européen des brevets**

(19)

(11) Veröffentlichungsnummer: **0 002 831**
**B1**

(12) **EUROPÄISCHE PATENTSCHRIFT**

(21) Anmeldenummer: **78101855.1**

(22) Anmeldetag: **28.12.78**

(51) Int. Cl.³: **A 61 K 6/08**

(54) **Opakes, photopolymerisierbares Zahnfüllmaterial**

(30) Priorität: **03.01.78 CH 38/78**

(43) Veröffentlichungstag der Anmeldung:
**11.07.79 Patentblatt 79/14**

(45) Bekanntmachung des Hinweises auf die Patenterteilung:
**03.09.80 Patentblatt 80/18**

(84) Benannte Vertragsstaaten:
**BE DE FR GB IT NL SE**

(56) Entgegenhaltungen:
**CH - A - 168 872**
**DE - A - 2 126 419**
**DE - A - 2 646 416**
**FR - A - 765 693**
**US - A - 4 046 578**
**G. R. Harrison et al.: "Practical spectroscopy",**
**1948,**
**Prentice Hall Inc. New York, Seiten 52—53**

(73) Patentinhaber: **Espe Fabrik pharmazeutischer**
**Präparate GmbH**
**D - 8031 Seefeld**
**Obb DE**

(72) Erfinder: **Schmitt, Werner, Dr.**
**Prinzenweg 10**
**D - 8130 Starnberg DE**
**Purrmann, Robert, Dr.**
**Riemerschmidstrasse 18**
**D - 8130 Starnberg DE**
**Jochum, Peter, Dr.**
**Pointweg 5**
**D - 8031 Hechendorf a. Pilsensee DE**
**Hübner, Heinz-Joachim, Dr.**
**Friedingerstrasse 14**
**D - 8031 Seefeld**
**Obb. DE**

(74) Vertreter: **Wuesthoff, Franz, Dr.-Ing.**
**Patentanwälte Wuesthoff-v. Pechmann-Behrens-**
**Goetz Schweigerstrasse 2**
**D - 8000 München 90 DE**

# 0 002 831

## Opakes, photopolymerisierbares Zahnfüllmaterial

Zahnfüllmaterial auf Basis von ungesättigten Polyestern, z. B. Diacrylat- oder Dimethyacrylatesterverbindungen das durch Licht in Gegenwart bestimmter Polymerisationsinitiatoren in situ schnell ausgehärtet werden kann, ist seit langem bekannt. So sind in der GB-A-569 974 photopolymerisierbare Dentalmassen für Zahnfüllungen beschrieben, die aus einem Gemisch aus Polyacrylaten und Acrylatestermonomeren bestehen und als Photoinitiator Benzoin enthalten. Durch eine Bestrahlung mittels einer UV-Lichtquelle werden diese Massen dann nach Applizierung im Munde gehärtet. Auch in der DE-A-21 26 419 bzw. der entsprechenden US-A-3 709 866, ebenso wie in der DE-A-2 315 645 sind solche durch UV-Licht polymerisierbaren Dentalmassen beschrieben, die noch zusätzliche Polymerisationskatalysatoren enthalten, um die schnelle und vollständige Aushärtung zu erreichen. Durch bestimmte Aktivatoren, insbesondere aliphatische oder aromatische bzw. gemischte aliphatisch-aromatische Phosphite kann die Lichtempfindlichkeit der polymerisierbaren Massen auf Basis von Acrylsäureestern noch gesteigert werden, wie dies in der DE-A-26 46 416 offenbart ist.

Um ein sichere und insbesondere vollständige Polymerisation und Aushärtung der Zahnfüllungen zu erreichen, muß das durch die Bestrahlung in situ härtbare Material eine gute Durchlässigkeit für kurzwelliges Licht besitzen, damit die Strahlen nicht nur in den Oberflächenbereich, sondern auch tief in die Masse bis zu ihrer rückwärtigen Oberfläche eindringen können, um auch dort die Polymerisation in gleicher Intensität zu initiieren, wie dies an der der Strahlenquelle zugewandten Oberfläche der Füllungen der Fall ist.

Diese gute Lichtdurchlässigkeit der Zahnfüllmassen hat nun aber kosmetische Nachteile, wenn sie im Frontzahnbereich für Approximalkavitäten, deren palatinale Wand nicht mehr vorhanden ist, Verwendung finden, da dann dort diese durchscheinenden Füllungen infolge des dunklen Hintergrunds der Mundhöhle dunkel erscheinen. Eine ähnliche Problematik ergibt sich bei Füllungen dünner Schichtdicke mit nicht zahnfarbenem Hintergrund, z. B. mit durchscheinender Unterfüllung. Ersichtlich ergibt sich hier nun ein schwieriges Problem bei den durch Licht härtenden Zahnfüllmassen. Wenn man nämlich aus kosmetischen Gründen für die Füllungen an diesen Stellen ein weißpigmentiertes Material, z. B. eine mit Lithopone, Titandioxid bzw. Zinkoxid gefüllte Masse verwendet, wird nur eine dünne Oberflächenschicht des Zahnfüllmaterials bei der Bestrahlung mit Licht gehärtet, weil diese Pigmente nicht nur das sichtbare, sondern mehr oder weniger stark auch die Strahlung im UVA- und UVA-nahen Lichtbereich (ca. 320—500 nm) absorbieren. Infolgedessen muß man diese Füllung schichtweise aufbauen und nach jedem dünnen Schichtauftrag erneut die Härtung durch Bestrahlung durchführen Hierdurch wird aber der Zahnarzt stark belastet und die Behandlung so sehr verzögert, daß dadurch die Vorteile des photohärtbaren Zahnfüllmaterials bei diesen Anwendungen im Frontbereich der Zähne zu nichte gemacht werden. Sie konnten sich daher für diese seitlichen Zahnfüllungen im Frontbereich des Gebisses bisher nicht durchsetzen.

Es wurde nun uberraschenderweise festgestellt, daß eine ausreichend tiefgreifende Härtung dann erreicht werden kann, wenn man els pigmentierenden Füllstoff für diese photohärtbaren Dentalmassen fein zerteiltes Calciumfluorid verwendet. Dieses Pigment wird erfindungsgemäß bei dem Zahnfüllmaterial in einer Menge von 1—20 Gew.-%, vorzugsweise 2—15 Gew.-%, bezogen auf die Füllungsmischung, verwendet. Calciumfluorid ist auch physiologisch unbedenklich. Die Teilchengröße soll vorzugsweise unter 60 $\mu$m liegen; besonders geeignet ist Calciumfluorid $<10$ $\mu$m. Gegebenenfalls kann auch zusätzlich zur Vermeidung von Absetzerscheinungen im Füllungsmaterial noch fein verteilte, sogenannte kolloidale Kieselsäure als Verstärkungsmittel vorhanden sein, da dieses reine Siliciumoxidmaterial bekanntlich UV-lichtdurchlässig ist, was auch mehr oder weniger für den sichtbaren Bereich gilt, wenn es fein genug und gleichmäßig in der Harzmasse verteilt ist. Die Massen können ferner die üblichen Füllstoffe auf Basis von $SiO_2$ oder Silikaten enthalten.

Durch den pigmentierenden erfindungsgemäß zu verwendenden Füllstoff ist das Zahnfüllmaterial ausreichend durchlässig für kurzwelliges Licht, welches die Härtung in Verbindung mit den Photosensibilisatoren und gegebenenfalls vorhandenen Aktivatoren initiiert. und besitzt dennoch eine ausreichende Absorptions-bzw. Reflektionsfähigkeit für die Strahlen des sichtbaren Teils des Lichtspektrums, so daß das oben erwähnte dunkle Durchscheinen bei ihrer Verwendung nicht auftritt, sondern das Zahnfüllmaterial die übliche mattweiße Färbung der Zähne besitzt.

Zur Polymerisation der erfindungsgemäßen Zahnfüllmaterialien werden die herkömmlichen Photoinitiatoren verwendet, wie z. B. Benzoin und seine Derivate, Benzilmonoketale und 1,2-Diketone. Um eine schnellere Aushärtung dieser auf UV-Strahlung und/oder den kurzwelligen Anteil des sichtbaren Lichts (UVA und UVA-nahe Strahlung) reagierenden Massen zu erzielen, können ferner organische Phosphite oder tertiäre Amine zugesetzt werden.

Zum Nachweis der besonderen Wirkung des erfindungsgemäßen Weißpigmentzusatzes wurden Vergleichsversuche durchgeführt. Es wurde hierbei unter Venwendung sonst identischer Bestandteile des Zahnfüllmaterials jeweils so viel Weißpigment zugegeben, daß die Polymerisationstiefe (Schichtdicke) bei einer Belichtung während 20 Sekunden mit einem Standard UV-Polymerisationsgerät (Uviolite) stets 3,2 $\pm$ 0,1 mm betrug.

**0 002 831**

Diese Schichtdickenbestimmung erfolgte in der Weise, daß ein Ring mit einer lichten Weite von 5 mm und einer Dicke von 5 mm mit der zu polymerisierenden Masse beschickt wurde; zur Abdeckung der Oberflächen wurden jeweils dünne Glasplatten verwendet. Für alle Versuche wurde folgende Standardmischung verwendet:

TABELLE I

| Substanz | Gew.-Teile |
| --- | --- |
| 2,2-Bis-[p-($\gamma$-hydroxy-propoxy-)phenyl]-propan-dimethacrylat | 100 |
| p-Methoxy-phenol | 0,02 |
| Benzil-dimethylketal | 0,5 |
| Didecyl-phenyl-phosphit | 0,5 |
| Mikrofeine, pyrogene Kieselsäure (silanisiert) | 5 |
| Quarz (<60 $\mu$) zahnfarben pigmentiert (silanisiert) | 420 |

Die Polymerisation erfolgt mit dem UV-Gerät in der Weise, daß der Quarzstab, an dessen Ende die UV-Strahlen austreten, unmittelbar auf die obere Glasplatte senkrecht aufgesetzt wurde. Anschließend wurde die Dicke der polymerisierten Schicht gemessen. Durch mehrere Versuche wurde dann die Menge an dem jeweiligen Pigment ermittelt, die den gleichen Wert der Polymerisationstiefe von ca. 3,2 mm unter sonst gleichen Bedingungen ergibt. Die Mengen der zu dieser Paste jeweils zugegebenen Weißpigmente sind in Spalte 2 der folgenden Tabelle II aufgeführt. Für die Transparenzmessung wurden jeweils runde Plättchen mit einem Durchmesser von 14,5 mm und einer Dicke von 0,6 mm verwendet. Zur Messung der Lichtdurchlässigkeit diente das Dr.-Lange-Transparenzmessgerät, mittels dessen der Anteil des durchgehenden sichtbaren Lichts bestimmt werden kann. In der Tabelle II finden sich diese Angaben in Prozent in der letzten Spalte.

TABELLE II

| Weißpigment | % auf Paste | Lichtdurchlässigkeit |
| --- | --- | --- |
| Lithopone | 0,08 | 13,6% |
| Titanweiß R 25 (Kronos) | 0,05 | 17,9% |
| Calciumfluorid (Merck) | 10 | 3,0% |
| Zinkoxid G 6 | 0,017 | 29,1% |
| Zinndioxid (Merck) | 0,32 | 9,2% |

Trotz der zum Teil mehr als 100-fachen Menge an Pigment wird bei dem erfindungsgemäßen Stoff die gewünschte Härtung erreicht. Aus der letzten Spalte der Tabelle II ist zu ersehen, daß bei einer Polymerisationstiefe von jeweils 3,2 mm die Durchlässigkeit für sichtbares Licht bei Verwendung der erfindungsgemäßen Substanz Calciumfluorid nur etwa 1/4—1/6 derjenigen beträgt, die sich mit den üblicherweise verwendeten Weißpigmenten Lithopone oder Titanweiß ergibt. Diese Tatsache steht in Übereinstimmung mit der Beobachtung, daß bei den erfindungsgemäßen Massen sogar bei dünnen Füllungen das oben erwähnte kosmetisch unerwünschte dunkle Durchscheinen der Mundhöhle nicht mehr beobachtet wird.

3

# 0 002 831

## Beispiel 1

Es wird ein Gemisch hergestellt, enthaltend

| Substanz | Gew.-Teile |
| --- | --- |
| 2,2-Bis-[p-($\gamma$-hydroxy-propoxy-)phenyl]-propan-dimethacrylat | 100 |
| p-Methoxy-phenol | 0,02 |
| Benzil-dimethylketal | 0,5 |
| Didecyl-phenyl-phosphit | 0,5 |
| Mikrofeine, pyrogene Kieselsäure (silanisiert) | 5 |

42 g dieses Gemischs werden dann mit 189 g zahnfarben pigmentiertem Quarz ($<$60 $\mu$m), der in üblicherweise silanisiert ist sowie mit 11 g feinkörnigem $CaF_2$ zu einer Zahnfüllmischung verknetet.

Durch Bestrahlen mit dem UV-Polymerisationsgerät Uviolite erhält man Polymerisate, die eine Druckfestigkeit von 350 MPa (3600 kp/cm$^2$) aufweisen. Zahnfüllungen, die aus dieser Masse hergestellt werden, sind kosmetisch hervorragend; auch bei durchgehenden Füllungen im Frontzahnbereich erscheinen sie völlig zahnähnlich.

## Beispiel 2

Es wird ein Gemisch hergestellt, enthaltend

| Substanz | Gew.-Teile |
| --- | --- |
| 2,2-Bis-[p-($\gamma$-hydroxy-propoxy-)phenyl]-propan-dimethacrylat | 100 |
| p-Methoxy-phenol | 0,02 |
| $\alpha$-Methyl-benzoin-methyläther | 0,5 |
| Dioctylphosphit | 0,8 |
| Mikrofeine, pyrogene Kieselsäure (silanisiert) | 5 |

Man verfährt weiter wie in Beispiel 1 und erhält so eine Zuhnfüllmasse, deren Konsistenz für die Herstellung von Zahnfüllungen und für Aufbauten fehlender Zahnteile hervorragend geeignet ist.

Die damit gelegten Füllungen sind auch im Frontzahnbereich ausreichend opak und kosmetisch einwandfrei.

## Beispiel 3

Es wird ein Gemisch hergestellt, enthaltend

| Substanz | Gew.-Teile |
| --- | --- |
| Bis-hydroxymethyl-tricyclo [5.2.1.0$^{2,6}$]-decan-diacrylat | 100 |
| Jonol | 0,02 |
| p-Methoxyphenol | 0,02 |
| Phenanthrenchinon | 0,05 |
| N,N-Dimethylethanolaminmethacrylat | 4 |
| Mikrofeine, pyrogene Kieselsäure (silanisiert) | 5 |

4

Ferner wird eine Pulvermischung hergestellt aus 179,5 g silanisiertem zahnähnlich eingefärbtem Quarz (<60 $\mu$m) und 9,1 g feinkörnigem Calciumfluorid. Man verknetet 4 g des flüssigen Gemischs mit 18 g der Pulvermischung und erhält so eine Zahnfüllmasse, die mit dem kurzwelligen Anteil des sichtbaren Lichts erhärtet werden kann.

Damit hergestellte Aufbauten fehlender Zahnteile und Füllungen im Front-Zahnbereich sind nach dem Aushärten zahnähnlich opak und kosmetisch einwandfrei.

## Beispiel 4

Ein flüssiges Gemisch wird hergestellt aus

| Substanz | Gew.-Teile |
|---|---|
| Bis-hydroxymethyl-tricyclo [5.2.1.0$^{2,6}$]-decan-diacrylat | 100 |
| p-Methoxyphenol | 0,02 |
| Jonol | 0,02 |
| Benzildimethylketal | 0,6 |
| Didecyl-phenyl-phosphit | 0,5 |
| Mikrofeiner, pyrogener Kieselsäure (silanisiert) | 15 |

Weiterhin wird eine Pulvermischung hergestellt aus 153 g silanisiertem, zahnähnlich eingefärbtem Bariumglas (<60 $\mu$m; 25 Gew.-% BaO) und 5 g feinkörnigem Calciumfluorid.

Man verknetet 10 g des flüssigen Gemischs mit 41 g der Pulvermischung.

Mit der erhaltenen Zahnfüllmasse gelegte Füllungen und Zahnaufbauten sind röntgenopak und durch ihre Opazität auch für den Frontzahnbereich kosmetisch bestens geeignet.

## Beispiel 5

Ein flüssiges Gemisch wird hergestellt aus:

| Substanz | Gew.-Teile |
|---|---|
| Bis-hydroxymethyl-tricyclo- [5.2.1.0$^{2,6}$]-decan-dimethacrylat | 100 |
| Jonol | 0,02 |
| p-Methoxyphenol | 0,02 |
| Benzildimethylketal | 0,3 |
| Didecyl-phenyl-phosphit | 0,5 |
| Mikrofeiner, pyrogener Kieselsäure (silanisiert) | 5 |

Ein Heißpolymerisat, bestehend aus 40 Gew.-Teilen Poly(bis-hydroxymethyl-tricyclo[5.2.1.0$^{2,6}$]-decan-dimethylacrylat) und 60 Gew.-Teilen mikrofeiner, pyrogener, silanisierter Kieselsäure wird gemahlen, gesiebt und zahnähnlich eingefärbt.

11,2 g dieses Füllstoffs werden mit 0,8 g Calciumfluorid vermengt und mit 5 g des flüssigen Gemischs zu einer Zahnfüllmasse verknetet.

Diese Zahnfüllmasse ergibt nach dem Aushärten durch Bestrahlung polierfähige, zahnähnlich opake Füllungen, die auch im Frontzahnbereich kosmetisch hervorragend geeignet sind.

**Patentansprüche**

1. Opakes, photopolymerisierbares Zahnfüllmaterial auf Basis von Acrylsäureesterverbindungen enthaltend mono- und/oder polyfunktionelle Acryl- oder Methacrylesterverbindungen, Photo-Initiatoren, Pigmente sowie gegebenenfalls Aktivatoren und Siliciumdioxid- oder Silikat-Füllmittel, dadurch gekennzeichnet, daß es als Weißpigment fein verteiltes Calciumfluorid in einer Menge von 1—20 Gew.-%, bezogen auf die Füllungsmischung, enthält.

2. Zahnfüllmaterial nach Anspruch 1, dadurch gekennzeichnet, daß das Weißpigment in einer Menge von 2—15 Gew.-% enthalten ist.

3. Zahnfüllmaterial nach Anspruch 1 oder 2, dadurch gekennzeichnet, daß die Teilchengröße des Weißpigments weniger als 60 $\mu$m beträgt.

## Claims

1. An opaque, photopolymerizable dental filling material on the basis of acrylic acid ester compounds comprising mono- and/or polyfunctional acrylic or methacrylic ester compounds, photoinitiators, pigments and optionally activators and silicon dioxide or silicate fillers characterized in that it contains a finely divided calcium fluoride as white pigment in an amount of from 1—20% by weight, based on the filling mixture.

2. Dental filling material according to claim 1 characterized in that the white pigment is contained in an amount of from 2—15% by weight.

3. Dental filling material according to claim 1 or 2 characterized in that the particle size of the white pigment is less than 60 $\mu$m.

## Revendications

1. Produit opaque photo-polymérisable pour plombages dentaires à base d'esters d'acide acrylique, contenant des esters acryliques ou méthacryliques mono- et(ou) polyfonctionnels, des photoinitiateurs, des pigments et éventuellement des activateurs et des matières de charge à base de dioxyde de silicium ou de silicates, caractérisé en ce qu'il contient comme pigment blanc du fluorure de calcium finement divisé en une proportion comprise entre environ 1 et 20% en poids par rapport au mélange de matière obturatrice.

2. Produit pour plombages dentaires selon la revendication 1, caractérisé en ce que la proportion de pigment blanc est comprise entre environ 2 et 15% en poids.

3. Produit pour plombages dentaires selon l'une des revendications 1 et 2, caractérisé en ce que les particules du pigment blanc possèdent une granulométrie inférieure à environ 60 microns.